Europäisches Patentamt

European Patent Office

Office européen des brevets

(11) Publication number: **0 127 259**
**B1**

# EUROPEAN PATENT SPECIFICATION

(45) Date of publication of patent specification: **07.01.88**

(51) Int. Cl.⁴: **A 61 B 5/08,** G 01 N 1/00

(21) Application number: **84301149.5**

(22) Date of filing: **22.02.84**

(54) Throw-away breath sample device.

(30) Priority: **23.05.83 US 496792**

(43) Date of publication of application:
**05.12.84 Bulletin 84/49**

(45) Publication of the grant of the patent:
**07.01.88 Bulletin 88/01**

(84) Designated Contracting States:
**BE DE FR GB IT NL SE**

(56) References cited:
**DE-A-2 759 119**
**FR-A-1 209 769**
**GB-A-1 371 300**
**US-A-3 196 689**
**US-A-3 420 224**
**US-A-3 635 214**
**US-A-4 291 704**

(73) Proprietor: **Drägerwerk Aktiengesellschaft**
**Moislinger Allee 53-55**
**D-2400 Lübeck 1 (DE)**

(72) Inventor: **Metzger, Louis G.**
**One Piermont Road**
**Closter New Jersey (US)**

Courier Press, Leamington Spa, England.

## Description

### Background and statement of the invention

A throw-away device is provided for taking breath samples. More particularly, the invention is directed to a throw-away device which will take a breath sample and hold it for a subsequent connection to an analyzer instrument for testing the sample. The device is of simplified construction which may be easily manufactured of inexpensive materials so that it may be used only once or several times prior to discarding. The device is particularly appropriate for use in determining carboxyhemoglobin in blood.

In the US—A—3 420 224 there is described and shown a throw-away apparatus for sampling respired air into a bag, especially suited for a subsequent analysis of carbon dioxide content of the expired air.

To this purpose, air is being breathed into and out of the bag by means of a mouthpiece, which is in sideways communication with a cylinder in which a piston is movable along its axis. One end of the cylinder is connected to the opening of the bag so as to give way to the respired air, when the piston is taken away from the opening and to block off the passageway of the mouthpiece to the bag, when the piston is put forward to seal the opening.

It is now well known and recognized by public health authorities that a relationship exists between the carboxyhemoglobin content of human blood, and the carbon monoxide of the alveolar air in the breath of humans. Alveolar air is the air contained in the deepest part of the lung. It is obtained by causing a person to inhale deeply, to hold the inhaled breath for a short while, and to cause that person to slowly exhale while catching or collecting the very last portion of the air with the lungs emptied. The device of the invention is arranged to take the sample of air exhaled, and to hold it for subsequent application to instrumentation for reading the breath sample. By doing so, the carbon monoxide content of the sample is determined, and the carboxyhemoglobin (COHb) content of the blood of the person from whom the sample is taken may be determined, rapidly, according to the formula.

$$\% \ COHb = \sqrt{109.08 + 7.600_A} \cdot 11.89.$$

A patient, using the 20 second breath-holding technique, blows into the instrument of the invention which collects and holds the sample for subsequent application to an instrument, as described below, for an analysis of the carbon monoxide content. This is converted into the percent of carboxyhemoglobin according to this well established relationship.

The arrangement of the invention here allows for taking samples of within the range of 500—800 ccs (cubic centimeters). The sample is held for subsequent connection to an instrument for reading the sample taken. Since only 200 ccs are needed to take a reading on the instrumentation, two to three separate subsequent readings may be taken from a single sample exhaled from a patient. Thus, an immediate reading can be taken of a patient in only a few seconds, rather than waiting for a blood test result. This is particularly important for people exposed to high carbon monoxide concentrations such as those involved in fire-fighting, for example. The device is particularly appropriate for use by paramedics or emergency room personnel for determining and preventing hypoxic stress in such people exposed to high carbon monoxide concentrations.

The apparatus of the invention involves a device which is particularly appropriate for use in emergency situations because a breath sample can be taken under unusual and difficult conditions, and the sample held for subsequent application to an instrument. Thus, a paramedic may take a sample from an individual and the sample is held until the paramedic is able to unpack an instrument which may be included in his gear for a subsequent reading. It is not necessary that a difficult and involved instrument must be connected to or held adjacent to the person from whom the sample is being taken. Once the device containing the sample is connected to the instrumentation, an arrangement is provided for such a connection, in accordance with a specific embodiment of this invention, which provides an automatic sample release of the sample, and a controlled feeding of the sample to the instrumentation so that the appropriate quantity of air sample is fed at a constant rate to the instrument for the reading. The proposed apparatus is particularly suited for mass screening as performed by health authorities, in that it allows collecting samples by several technicians who, after mass collection wil introduce, one by one the identified samples into the associated reading instrument.

Other objects and advantages of this invention will be apparent from the following description, the accompanying drawings and the appended claims.

### Description of the drawings

Fig. 1 is a partial vertical sectional view of a device illustrating the invention applied to the lips of a patient from who a sample is being taken;

Fig. 2 is a partial vertical sectional view of the device of Fig. 1 with an additional separate mouthpiece being utilized; and

Fig. 3 is a partial vertical sectional view of the device of Fig. 1 attached to the sample collector and valve release block of the invention which serves to feed a sample to instrumentation at a constant rate for a reading.

### Detailed description of the invention

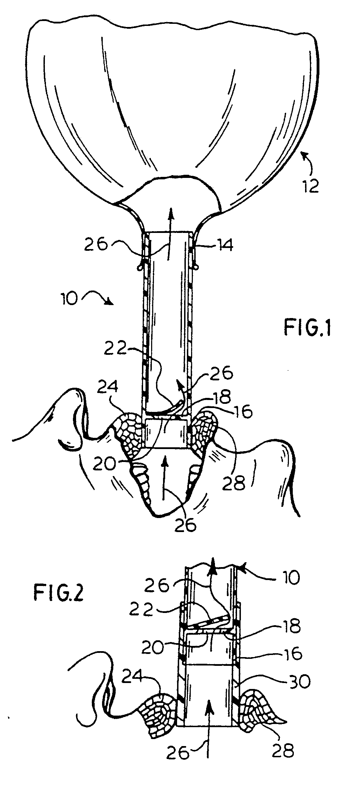

Referring to the drawings in which like reference characters refer to like parts throughout the several views thereof, a breath collector tube 10 is shown in Fig. 1 having a ballon 12 fitted on the end 14 thereof for receiving a breath sample.

As shown in Fig. 1, the lips 24 of the mouth 28 of a patient receives the end 16 of the breath collector tube 10. The patient blows in the direction of arrows 26 the breath sample, as described above, which is the last portion of an inhaled and held sample of breath which passes according to arrows 26 into balloon 12 through an orifice 20. The orifice 20 is positioned in valve seat 18, and is controlled by a valve flap 22, shown in its open position in Fig. 1. The valve flap 22, as will be understood, opens to the position shown in Fig. 1, under the force of the sample blown in the direction of arrows 26 by the patient through the breath collector tube.

For sanitary reasons, the breath collector tube may have a separate disposable mouthpiece 30, as shown in Fig. 2, which mouthpiece may be removed from the breath collector tube 10 after each sample taking, and discarded for sanitary reasons. Under extreme conditions of contagen or in order to comply with local laws concerning contamination, it may be necessary to discard the entire apparatus, after the taking and measuring of each individual sample in breath collector tube 10 received in the balloon 12. Since the collector arrangement is of simplified construction and may be comprised of inexpensive materials, discarding after each use is not a serious problem in use. The balloon may contain 500—800 cc of air in the form of a breath sample taken from a patient. The sample, once taken may be held in the device shown in Figs. 1 or 2 until such time as the device is connected to instrumentation for taking a reading of the sample.

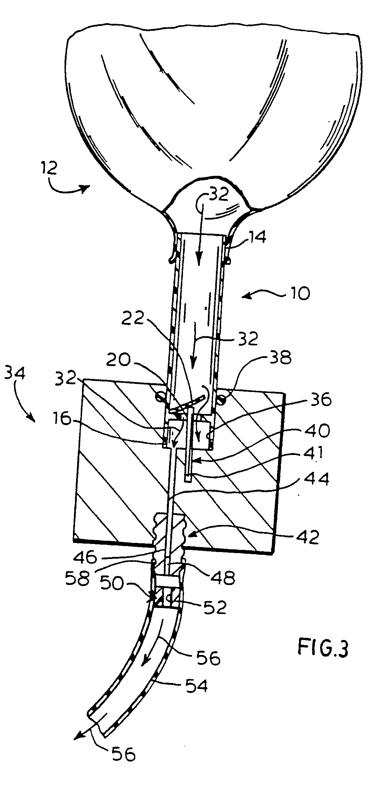

The arrangement for receiving and reading the sample taken is shown in Fig. 3. In Fig. 3 a sample collector and valve release block 34 is shown having a bore 36 in the top surface thereof. Bore 36 may be of a diameter just slightly larger than the outer diameter of breath collector tube 10 for receiving end 16 of breath collector tube 10 in a sealing engagement therein. In O-ring seal 38 is incorporated into bore 36 for maintaining this sealing engagement between bore 36 and the breath collector tube 10. Positioned in the bottom of bore 36 is a fixed pin 40, which is held in place in a counterbore 41 in the bottom surface of bore 36. Pin 10 extends upwardly into bore 36 for engaging the valve flap 22 when the breath collector tube 10 is received in bore 36. Thus, valve 22 is opened automatically when the breath collector tube 10 is received in bore 36.

As can be seen in Fig. 3, a passage 44 is formed in valve release block 34 for carrying a sample in the direction of arrows 32 from the ballon 12, containing the sample, through the breath collector tube 10, through orifice 20 when valve 22 is opened. In the bottom of block 34 at the end of passage 44 opposite bore 36 is a threaded bore 42 for receiving a threaded fitting 46. Fitting 46 also includes a passage 48 which cooperates the passage 44 in block 34 for providing flow communication from bore 36 to a flexible tube 54. The end 58 of tube 54 is fitted over the fitting 46 for containing the sample and directing the sample in the direction of arrows 56 toward an instrument for reading the sample. The instrument may be, for example, a portable carbon monoxide monitor ECOLYZER[R] Model Number 210 or 211 manufactured by Energetics Science Division of Becton Dickinson and Company, Six Skyline Drive, Hawthorne, New York 10532. Positioned in flexible tube 54 may be a flow restrictor 50 with a controlled orifice 52 so as to maintain a controlled continuous rate of flow of the sample in the direction of arrows 56 to the instrument for the reading of the sample.

As discussed above, since balloon 12 may have a capacity of 500—800 cc, a sample may provide two or three separate readings in the attached instrument. That is, the Model 210 or 211 ECOLYZER[R] requires only 200 cc to obtain a reading within the range of 30 seconds to one minute. Thus, three separate readings from a single sample may be taken within a very short period of time in order, for example, for a paramedic to obtain information concerning possible over exposure to carbon monoxide in an emergency situation. It will be appreciated, that such rapid determinations can also be made by hospital personnel so that they do not need to wait for a testing of a blood sample prior to making a determination of such exposure. It will be appreciated, that it is within the purview of of this invention that passage 44 in block 34 or passage 48 in fitting 46 may be sized and configured to provide a restricted controlled flow rather than the use of a separate flow restrictor 50 as shown in Fig. 3.

Thus, as will be appreciated from the foregoing, there is provided in accordance with this invention a simple inexpensive throw-away light-weight type device for obtaining breath samples for determining the carbon monoxide content of the sample. Moreover, the arrangement is so simple and light-weight that it may be utilized in very difficult situations requiring emergency readings and so forth. The apparatus of the invention is arranged to take and hold a sample until it can be properly attached to instrumentation for a reading. Thus, it is not necessary for the user to be burdened with instruments at the site where the sample is being taken.

The breath collector tube of the invention may be comprised of metal, plastic or carboard. Preferably, the throw-away disposable mouthpiece 30 will be comprised of carboard which may be used and thrown away. The collector tube, preferably, has a length of about three to six inches and a diameter of about one-half inch. The valve release block 34 may vary in size, as will be appreciated as long as it is able to accommodate a bore 36, pin 40 and counterbore 41, and a separate bore 42 as shown in Fig. 3. Preferably, bore 36 has a length of about three times the diameter of tube 10 and a diameter of just slightly larger than the diameter of tube 10.

Orifice 52 is of a size which will allow for a specified quantity of a sample of about 200 cc to pass therethrough within a period of about 30 seconds. As stated above, passage 44 of passage 48 may be sized and configured to provide this restricted and controlled flow. Moreover, the orifice 20 in valve seat 18 may also be arranged to provide a controlled restricted flow. While a flapper valve of a soft flexible material, such as 22 is shown and described, it will be understood that other configurations of one-way valves may be utilized for receiving and holding a breath sample until such time as the sample is to be taken and measured. Such valves include, for example, spring biased valves.

While the forms of apparatus herein described constitute preferred embodiments of the invention, it is to be understood that the invention is not limited to these precise forms of apparatus, and that changes may be made therein without departing from the scope of the invention which is defined in the appended claims.

### Claims

1. A throw-away apparatus for taking and holding breath samples, comprising
(a) an elongate hollow body (10) defining a chamber therein:
(b) an inlet at one end (16) of the body, the inlet defining a mouthpiece for the apparatus;
(c) an outlet at the end (14) of the body opposite the inlet;
(d) expandable sample container means (12) in flow communication with the outlet for receiving breath samples therefrom, characterized by
(e) a valve orifice (20) adjacent the inlet; and
(f) one-way valve means (22) for closing the orifice (20);
(g) the valve means being biased to allow passage of breath sample from the inlet to the outlet.

2. An apparatus according to claim 1, further characterized by a hollow elongate throw-away mouthpiece (30) for the breath sample apparatus, the mouthpiece having first and second open ends at least one of which is of a size for receiving in press-fit engagement the inlet end (16) of the body inlet.

3. An apparatus according to claim 2, further characterized in that the body (10) is tubular; the mouthpiece is tubular; and the internal diameter of the mouthpiece is large enough to receive the tubular inlet end of the body in pressfit engagement.

4. An apparatus according to claim 1, 2 or 3, further characterized in that the one-way valve means is a resilient flap valve (22) mounted with one side edge connected to the body and the opposite edge mounted for movement between a closed position closing the orifice (20) to an open position.

5. An apparatus according to any preceding claim, further characterized in that the expandable sample container means (12) is a balloon.

6. An apparatus according to any preceding claim, further characterized by a breath sample collector therefor, the breath sample collector comprising
(a) a collector body (34);
(b) a sample collector body inlet for receiving the breath sample body inlet;
(c) abutment means (40) in the sample collector body inlet for engaging and biasing the one-way valve means to an open position;
(d) an outlet in the collector body;
(e) means connected to the outlet for conveying a breath sample therefrom to a breath sample testing instrument;
(f) means (44) providing flow communication from the sample collector body inlet to the breath sample conveying means, and
(g) flow restrictor means (50) in the flow communication means for controlling the rate of breath sample flow therethrough.

7. An apparatus according to claim 6, further characterized in that the breath sample body inlet is tubular; and the sample collector body inlet is a bore (36) having a diameter of a size for receiving the breath sample collector body inlet in sealing engagement.

8. An apparatus according to claim 6 or 7, further characterized in that the abutment means (40) is an elongated rod fixed in the sample collector body inlet; with the rod extending into the breath sample body inlet for engaging and opening the one-way valve means when the breath sample body inlet is received into the sample collector body inlet.

9. An apparatus according to claim 6, 7 or 8, further characterized by an O-ring (38) in the inlet bore of the sample collector body for enhancing sealing engagement with the breath sample body.

### Patentansprüche

1. Ein Einmal-Gerät zur Aufnahme und Bereithaltung von Atemgasproben, enthaltend:
(a) einen länglichen Hohlkörper (10), der eine in ihm befindliche Kammer definiert;
(b) einen Einlaß an dem einen Ende (16) des Hohlkörpers, wobei der Einlaß ein Mundstück für das Einmalgerät bildet; ·
(c) einen Auslaß an dem dem Einlaß gegenüberliegenden Ende (14) des Hohlkörpers;
(d) einen ausdehnbaren Probenbehälter, in Strömungsverbindung mit dem Auslaß gebracht, zur Aufnahme von Atemgasproben, gekennzeichnet durch
(e) eine Ventilöffnung (20), benachbart zum Einlaß;
(f) eine Ein-Weg-Ventilanordnung (22) zum Verschließen der Öffnung (20), wobei
(g) die Ventilanordnung derart vorgespannt ist, daß die Durchströmung der Atemgasprobe vom Einlaß zum Auslaß ermöglicht ist.

2. Einmal-Gerät nach Anspruch 1, dadurch gekennzeichnet, daß ein hohlförmiges, längliches Einmal-Munstück (30) für das Einmal-Gerät ein erstes und ein zweites offenes Ende besitzt, von denen zumindest eines von einer solchen Größe ist, daß es in einem Presspassungs-Eingriff das Einlaßende (16) des Hohlkörpereinlasses aufnimmt.

3. Einmal-Gerät nach Anspruch 2, dadurch gekennzeichnet, daß der Hohlkörper (10) und das Munstück (30) röhrenförmig sind, und daß der Innendurchmesser des Mundstückes groß genug ist, um das röhrenförmige Einlaßende (16) des Hohlkörpers in einem Presspassungs - Eingriff aufzunehmen.

4. Einmal-Gerät nach einem der Ansprüche 1 bis 3, dadurch gekennzeichnet, daß die Ein-Weg-Ventilanordnung ein elastisches Klappenventil (22) ist, das mit dem einen Seitenrand mit dem Hohlkörper verbunden, und mit dem dazu gegenüberliegenden Seitenrand bewegbar zwischen einer die Öffnung (20) verschließenden Stellung und einer die Öffnung freigebenden Stellung angeordnet ist.

5. Einmal-Gerät nach einem der Ansprüche 1 bis 4, dadurch gekennzeichnet, daß der ausdehnbare Probenbehälter (12) ein Ballon ist.

6. Einamal-Gerät nach einem der Ansprüche 1 bis 5, dadurch gekennzeichnet, daß ein Atemgasproben-Sammler vorgesehen ist, enthaltend

(a) ein Sammlergehäuse (34);

(b) einen Sammlergehäusseeinlaß zur Aufnahme des Einlasses des Atemgasprobenbehälters;

(c) eine Gegenspannvorrichtung (40) im Sammlergehäuseeinlaß, die an die Ein-Weg-Ventilanordnung angreift und sie in eine Öffnungsposition vorspannt;

(d) ein Auslaß im Sammlergehäuse;

(e) eine mit dem Aulaß verbundene Vorrichtung zur Beförderung der Atemgasprobe von dort zu einem Atemgas-Prüfgerät;

(f) Einrichtungen (44) zur Herstellung einer Strömungsverbindung zwischen dem Probensammler - Einlaß und der Beförderungsvorrichtung, und

(g) eine Strömungsbegrenzung (50) in der Strömungsverbindung zur Festlegung der Atemgasflowrate.

7. Einmal-Gerät nach Anspruch 6, dadurch gekennzeichnet, daß der Einlaß des Atemgasprobenbehälters röhrenförmig und der Einlaß des Atemgasprobensammlers eine Bohrung (36) mit einem solchen Durchmesser ist, daß er den Sammlereinlaß in dichtem Eingriff aufnimmt.

8. Einmal-Gerät nach Anspruch 6 oder 7, dadurch gekennzeichnet, daß die Gegenspannvorrichtung (40) ein länglicher Stift ist, der im Sammlereinlaß aufgenommen ist, wobei der Stift in den Einlaß des Atemgasprobenbehälters hineinragt und in die Ein-Weg-Ventilanordnung eingreift und sie öffnet, sobald der Einlaß des Atemgasprobenbehälters in den Einlaß des Sammlergehäuses aufgenommen ist.

9. Einmal-Gerät nach Anspruch 6, 7 oder 8, dadurch gekennzeichnet, daß ein O-Ring (38) in der Einlaßbohrung DES Sammlergehäuses zur Verbesserung der Dichtwirkung mit dem Atemgasprobenbehälter vorgesehen ist.

**Revendications**

1. Appareil jetable pour prélever er conserver des échantillons d'air respiratoire, comprenant:

(a) un corps creux oblong (10) définissant à l'intérieur une chambre;

(b) une entrée à une extrémité (16) du corps, l'entrée définissant un embout buccal pour l'appareil;

(c) une sortie à l'extrémité (14) du corps opposée à l'entrée;

(d) un contenant d'échantillons dilatable (12), en communication de flux avec la sortie pour recevoir des échantillons d'air respiratoire provenant de cette dernière; caractérisé par:

(e) un orifice de valve (20) au voisinage de l'entrée; et

(f) une valve antiretour (22) pour fermer l'orifice (20);

(g) la valve étant sollicitée pour permettre le passage de l'échantillon d'air respiratoire de l'entrée vers la sortie.

2. Appareil selon la revendication 1, caractérisé en outre par un embout buccal (30) jetable oblong et creux pour l'appareil d'échantillonnage d'air respiratoire, l'embout buccal présentant des première et seconde extrémités ouvertes dont au moins une d'entre elles est d'une taille lui permettant de recevoir en ajustement serré l'extrémité d'entrée (16) du corps.

3. Appareil selon la revendication 2, caractérisé en outre en ce que le corps (10) est tubulaire; l'embout buccal est tubulaire; et le diamètre intérieur de l'embout buccal est suffisamment grand pour recevoir en ajustement serré l'extrémité d'entrée tubulaire du corps.

4. Appareil selon la revendication 1, 2 ou 3, caractérisé en outre en ce que la valve antiretour est une valve à clapet élastique (22) montée avec un bord latéral assemblé au corps et le bord opposé monté mobile entre une position fermée obturant l'orifice (20) et une position ouverte.

5. Appareil selon une quelconque des revendications précédentes, caractérisé en outre en ce que le contenant d'échantillons dilatable (12) est un ballon.

6. Appareil selon une quelconque des revendications précédentes, caractérisé en outre par un collecteur d'échantillons d'air respiratoire destiné à cet appareil, le collecteur d'échantillons d'air respiratoire comprenant:

(a) un corps de collecteur (34);

(b) une entrée de corps de collecteur d'échantillons, destinée à recevoir l'entrée du corps de l'appareil d'échantillonnage;

(c) un moyen de butée (40) disposé dans l'entrée du corps de collecteur d'échantillons, destiné à solliciter la valve antiretour en position ouverte;

(d) une sortie dans le corps de collecteur;

(e) un moyen relié à la sortie pour transporter un échantillon d'air respiratoire de la sortie à un instrument de test d'échantillons d'air respiratoire;

(f) un moyen (44) assurant la communication de flux depuis l'entrée du corps de collecteur d'échantillons jusqu'au moyen de transport d'échantillons d'air respiratoire; et

(g) un moyen d'étranglement (50) dans le moyen de communication de flux, pour contrôler le débit d'écoulement de l'échantillon d'air respiratoire à travers ce dernier.

7. Appareil selon la revendication 6, caractérisé en outre en ce que l'entrée du corps de l'appareil d'échantillonnage est tubulaire; et l'entrée du corps du collecteur d'échantillons est un alésage (36) présentant un diamètre d'une taille lui permettant de recevoir en engagement étanche l'entrée du corps de l'appareil d'échantillonnage.

8. Appareil selon la revendication 6 ou 7, caractérisé en outre en ce que le moyen de butée (40) est une tige oblongue fixée dans l'entrée du corps du collecteur d'échantillons, la tige s'étendant dans l'entrée du corps de l'appareil d'échantillonnage pour s'engager avec la valve antiretour et ouvrir cette dernière lorsque l'entrée du corps de l'appareil d'échantillonnage est reçue dans l'entrée du corps du collecteur d'échantillons.

9. Appareil selon la revendication 6, 7 ou 8, caractérisé en outre par un joint torique (38) dans l'alésage d'entrée du corps de collecteur d'échantillons, pour améliorer l'engagement étanche avec le corps de l'appareil d'échantillonnage.

**0 127 259**

12

26 14

10

FIG.1

22

24 26

18

16

20

28

26

FIG.2

26

10

22

20

18

16

30

24

26

28

1

FIG.3